# EUROPEAN PATENT SPECIFICATION

(11) **EP 0 587 668 B1**
(45) Date of publication and mention of the grant of the patent: **23.08.1995**
(21) Application number: 92911532.7
(22) Date of filing: 27.05.1992
(51) Int. Cl.: A61M 11/02, A61M 15/08

(54) **NASAL DISPENSER ACTUATED BY NOSE CONTACT**
BEI KONTAKT MIT DER NASE AUSLÖSENDE NASALE VERABREICHUNGSVORRICHTUNG
DISTRIBUTEUR NASAL ACTIONNE PAR CONTACT AVEC LE NEZ

(30) Priority: 31.05.1991 DK 1031/91
(43) Date of publication of application: 23.03.1994
(73) Proprietor: BECHGAARD INTERNATIONAL RESEARCH AND DEVELOPMENT A/S, DK-2900 Hellerup (DK)
(72) Inventor: BECHGAARD, Erik, DK-2900 Hellerup (DK); CHRISTOFFERSEN, Peter, Bender, DK-2800 Lyngby (DK); HJORTKJAER, Rolf, Kuhlman, DK-3050 Humlebaek (DK); GIZURARSON, Sveinbjörn, IS-230 Keflavik (IS)
(74) Representative: Plougmann, Vingtoft & Partners A/S
(86) International application number: DK9200169
(87) International publication number: WO9221404

(56) References cited:
- EP-A- 0 452 728
- US-A- 2 052 321
- US-A- 3 269 389

## Description

The invention relates to a dispenser for intranasal administration of biologically active substances, hereafter designated as medicine doses. The dispenser is usable at mammals, including humans, in the following designated as patients.

Many illnesses, e.g. diabetes or epilepsy, have the result that the patient may suddenly feel indisposed or even faint. Such cases of indisposition may be remedied by quickly giving a medicine to the patient, e.g. glucagon to a hypoglycaemic diabetic or diazepam to an epileptic having an attack. Such medicine may be given by injection, but a nasal administration is preferred as it will often be an untrained person who has to help the patient.

For this use the patient himself may carry an emergency device by which a dose of medicine may quickly be administered.

WO 91/06333 discloses a device by which the air in a vessel may be compressed whereafter a nosepiece is inserted into a nostril and a manual trigger button is actuated to release the air from the vessel through the nosepiece. By actuating the trigger an aluminum membrane at each end of a cartridge is perforated and the air is led through this cartridge on its way to the nosepiece.

Such an apparatus is well usable for a patient who feels indisposed and knows that the medicine must be taken, but it is less suited as a first aid device which has to be handled by incidental people helping a patient who has fainted.

EP 0 452 728 relates to a device for trans nasal or oral application of a medicament and comprises a single finger-shaped applicator or a double finger-shaped applicator, the device being activated by pressing a button placed opposite to the dispensing end of the applicator in the direction towards the dispensing end and along the axis of the finger-shaped applicator. The activating button described in the reference is placed opposite to the nose when the device is used for nasal application.

The object of the invention is to provide a dispenser which needs only little or no instruction for use, is easy to use, and fast to handle.

This may be obtained by a dispenser comprising one or two nosepieces and being actuated by nose contact when the nosepiece or the nosepieces are inserted into a nostril or the nostrils of a mammal, e.g. a human patient.

According to the invention, the dispenser may further comprise a vessel containing a pressurized propellant, e.g. atmospheric air, at least one duct connecting the nosepiece or the nosepieces with the vessel, at least one valve closing the passage from the vessel to the nosepiece or the nosepieces, and means for opening the valve by nose contact, the biologically active substance being contained in the propellant or in the passage from the vessel to a nostril of the patient to be entrained with the propellant into his nose cavity when the valve or the valves are opened.

This dispenser is easy to use for an untrained person as all he has to do is to insert the nosepiece or the nosepieces into a nostril or the nostrils of a patient. When the nose piece or the nosepieces are fully inserted, the valves will be actuated and the propellant will be released to flow through the ducts and the nosepieces into the nose cavity of the patient, entraining the medicine placed in the passage for the propellant.

To make sure that the propellant is expelled quickly by a velocity securing the entraining of the medicine the valve may be of a bistable type being closed but turning to fully opened when activated.

According to the invention, the nosepiece or the nosepieces may be flexible to adapt themselves to the nose of the patient. The nosepieces may easily be forced away from each other or towards each other in accordance with the distance between the nostrils, and each nosepiece may be telescoped into itself to make sure that the valve or the valves may be activated from patients with short nostrils.

The medicine may be a liquid placed in the duct or the nosepiece or it may appear as a solid in the form of a powder and/or as a suspension placed in the duct and/or the nosepiece, and a solvent may be a part of the propellant or be placed in this duct or nosepiece upstream in relation to the powder or the suspension and be separated from this powder or suspension.

An incidental person may not know if one of the patient's nostrils is stopped, and, therefore, it is preferred that the dispenser has two nosepieces to make sure that the patient gets the needed medicine. Besides, the absorbing surface may be increased in this way.

According to a further development, each nosepiece may have its own independent duct and valve, the valves being designed to open simultaneously to omit that all the propellant is released through one valve before the other opens. Another way to omit such an uneven distribution of the propellant flowing through the two nosepieces may be to divide the vessel into two compartments each supplying its own independent duct, valve and nosepiece.

In a preferred embodiment having two nosepieces the valve or the valves are actuated by actuating means abutting the lower edge of the septum of the nostrils.

Although the dispenser may be designed to be reloaded with propellant and medicine, a disposable device is preferred. The device may be designed to change its appearance irreversibly when used e.g. by having a cover which cannot be replaced when it has been removed. The dispenser may be worn as an emergency kit which possibly will not be used at all during its lifetime. When used it may be disposed of and its change of appearance by use will make it evident that the device will no longer function and has to be replaced by a new one.

The medicine dose may be contained directly in the duct or the nosepiece or it may be contained in a capillary or in a cartridge inserted in the duct or the nosepiece. The volume of the dose may be of the order 0,1 »l - 50 ml, more preferred is 1 - 300 »l, and most preferred is 25 - 125 »l.

The invention will now be described in further details with reference to the drawings in which
Fig. 1 shows schematically an embodiment of a dispenser according to the invention,
Fig. 2 shows the dispenser of Fig. 1 in its released condition,
Fig. 3 shows another embodiment with a common valve for two nosepieces.

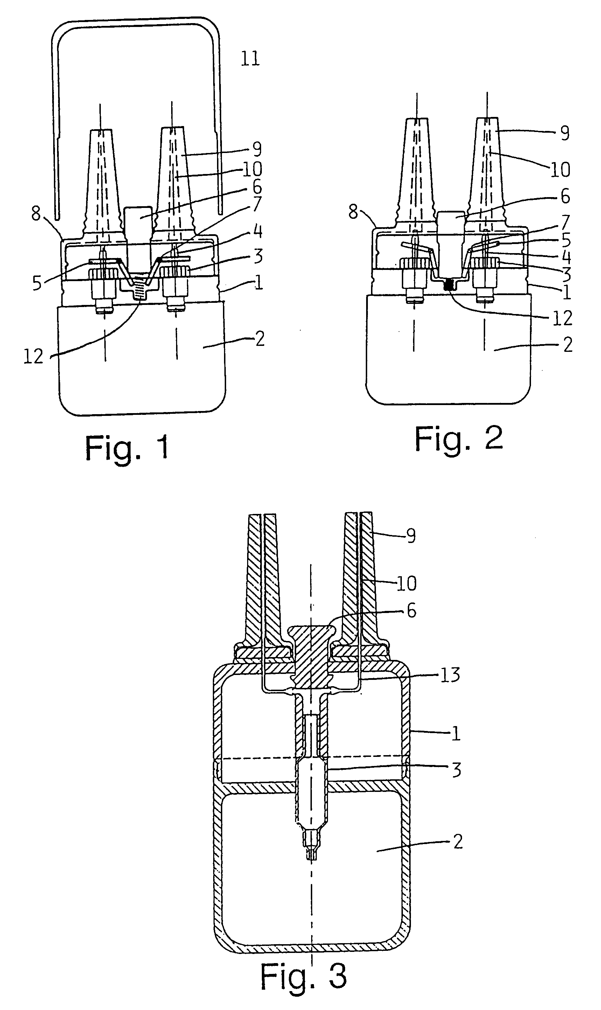

The dispenser in Fig. 1 comprises a housing 1 having a vessel 2 for containing a pressurized propellant, e.g. air under a superatmospheric pressure. In the upper wall of the vessel 2 a pair of valves 3 are mounted. These valves may be of the kind known from cigarette lighters having a hollow stem 4 which when lifted opens the valve for a flow through the central bore of the stem.

A lever 5 is at its one end gripping under a thickening on the stem 4 to lift this stem when its other end is actuated by the lower end of a button 6, the lever being about its middle mounted rotatably on a pin 7 dividing it into two arms.

On the top of the housing 1 a cover 8 is mounted having two nosepieces 9 each having an axial duct 10 containing a medicine dose to be dispensed. The medicine is confined in the ducts which at their bottom may be closed by a pierceable membrane and at their top may be closed by not shown stoppers which may be carried by a removable lid 11 covering the nosepieces when the device is not in use.

The button 6 is situated between the nosepieces 9 at the lower end thereof. The lower end of the button 6 faces an end of each of the levers 5, but is held out of engagement with these levers by a spring 12 pressing the button 6 upwards.

When the device is to be used the lid 11 is removed and thereby not shown stoppers carried by the lid are removed from the free ends of the nosepieces 9. The nosepieces are now passed into the nostrils of a patient until the septum of the nostrils abuts the upper end of the button 6. By further insertion of the nosepieces 9 the button 6 will be pressed down by the septum against the force of the spring 12 and its lower end will actuate one end of the respective levers 5 making the other ends lift the stems 4 of the respective valves releasing the pressurized propellant in the vessel 2 through these valves.

When the stems 4 of the valves are lifted, they will perforate the membranes at the bottom of the ducts 10 of the nosepieces, thereby ensuring that the released propellant will flow through these ducts and entrain the medicine in the ducts into the nostrils of the patient.

After use the device may be disposed of and to make it easy to see that the device is no longer usable provisions may be made to make it change its appearance irreversibly when it has been used. This may be done by making it impossible to replace the lid on a used device.

Fig. 3 shows a sectional view of another embodiment of the device. In this embodiment the two nosepieces are supplied with propellant through one common valve 3 which is opened in a not further specified way when the button 6 is pressed down by the septum of the nostrils when the nosepieces are passed into the nostrils of the patient. In this embodiment, it is further shown that the medicine doses are contained in capillary tubes 13 extending through the ducts in the nosepieces.

Although the shown embodiments have two nosepieces, devices having only one nosepiece, but with means for automatic release when the nosepiece is fully inserted in the nostril, are considered within the scope of the invention.

The same considerations go for embodiments where the device is not a disposable one, but may be reloaded with propellant and medicine. A metered dose device for repeated dosing wherein the medicine e.g. is dissolved or suspended in the propellant possibly together with a cosolvent is also considered within the scope of the invention.

## Claims

1. A dispenser for intranasal administration of one or several biologically active substances, e.g. medicine doses, comprising one or two nosepieces (9) and characterized in that it is actuated by nose contact when the nosepiece (9) or the nosepieces are inserted into a nostril or the nostrils of a mammal, e.g. a human.

2. A dispenser according to claim 1, characterized in that it further comprises a vessel (2) containing a pressurized propellant, e.g. atmospheric air, at least one duct (10) connecting the nosepiece or the nosepieces with the vessel (2), at least one valve (3) closing the passage from the vessel (2) to the nosepiece (9) or the nosepieces, and means for opening the valve by nose contact, the biologically active substance being contained in the propellant or in the passage from the vessel and through the duct in the nosepiece.

3. A dispenser according to claim 2, characterized in that the valve (3) or the valves are of a bistable type being closed but turning to fully opened when actuated.

4. A dispenser according to any of the preceding claims, characterized in that the nosepiece (9) or the nosepieces are flexible to adapt themselves to the nose of the patient.

5. A dispenser according to any of the preceding claims, characterized in that the biologically active substances are liquid or dissolved or a powder and/or a suspension and placed in the duct (10) or the nosepiece (9).

6. A dispenser according to any of the claims 1-4, characterized in that the biologically active substances appear as a solid in the form of a powder and/or as a suspension placed in the duct (10) and/or the nosepiece (9), and that a solvent is a part of the propellant or is placed in this duct (10) or nosepiece (9) upstream in relation to the powder or the suspension and is separated from this powder or suspension.

7. A dispenser according to any of the preceding claims, characterized in that it has two nosepieces (9).

8. A dispenser according to claim 7, characterized in that the nosepieces (9) each has its own independent duct (10) and valve (3), the valves being designed to open simultaneously.

9. A dispenser according to claim 7, characterized in that the vessel (2) is divided into two compartments each supplying its own independent duct (10), valve (3), and nosepiece (9).

10. A dispenser according to claim 7, 8 or 9, characterized in that the valve (3) or the valves are actuated by actuating means abutting the edge of the septum of the nostrils.

11. A dispenser according to any of the preceding claims, characterized in that it is designed as a disposable device.

12. A dispenser according to claim 11, characterized in that it is designed to irreversibly change its appearance when used.

13. A dispenser according to claim 12, characterized in that it is provided with a cover (4) which cannot be replaced when removed.

## Patentansprüche

1. Spender (1) zum intranasalen Verabreichen einer oder mehrerer biologisch aktiver Substanzen, beispielsweise Arznei-Dosen, mit einem oder zwei Nasenelementen (9), dadurch gekennzeichnet, daß der Spender durch Nasenkontakt betätigt wird, wenn das Nasenelement (9) oder die Nasenelemente in ein Nasenloch oder die Nasenlöcher eines Säugers, etwa einem Menschen, eingeführt werden.

2. Spender nach Anspruch 1, gekennzeichnet durch einen Behälter (2) mit einem unter Druck stehenden Treibmittel, etwa atmosphärischer Luft, mindestens eine Leitung (10), welche das Nasenelement oder die Nasenelemente mit dem Behälter (2) verbindet, mindestens ein den Kanal vom Behälter (2) zu dem Nasenelement (9) oder den Nasenelementen schließendes Ventil (3) und Mittel zum Öffnen des Ventils durch Nasenkontakt, wobei die biologisch aktive Substanz im Treibmittel oder im Kanal vom Behälter und über die Leitung in das Nasenelement enthalten ist.

3. Spender nach Anspruch 2, dadurch gekennzeichnet, daß das Ventil (3) oder die Ventile bistabil und geschlossen ist/sind, jedoch bei Betätigung vollständig geöffnet wird/werden.

4. Spender nach einem der vorstehenden Ansprüche, dadurch gekennzeichnet, daß das Nasenelement (9) oder die Nasenelemente flexibel sind, um sich an die Nase des Patienten anzupassen.

5. Spender nach einem der vorstehenden Ansprüche, dadurch gekennzeichnet, daß die biologisch aktiven Substanzen flüssig oder gelöst oder ein Pulver und/oder eine Suspension und in der Leitung (10) oder dem Nasenelement (9) plaziert sind.

6. Spender nach einem der Ansprüche 1-4, dadurch gekennzeichnet, daß die biologisch aktiven Substanzen als Festkörper in Form eines Pulvers und/oder als Suspension auftreten, welche in der Leitung (10) und/oder dem Nasenelement (9) angeordnet sind, und daß ein Lösungsmittel Teil des Treibmittels oder in dieser Leitung (10) oder diesem Nasenelement (9) stromaufwärts in Bezug auf das Pulver oder die Suspension angeordnet ist und von diesem Pulver oder dieser Suspension getrennt ist.

7. Spender nach einem der vorliegenden Ansprüche, dadurch gekennzeichnet, daß der Spender zwei Nasenelemente (9) aufweist.

8. Spender nach Anspruch 7, dadurch gekennzeichnet, daß die Nasenelemente (9) jeweils ihre eigene unabhängige Leitung (10) und ihr eigenes unabhängiges Ventil (3) aufweisen, wobei die Ventile simultan öffnen.

9. Spender nach Anspruch 7, dadurch gekennzeichnet, daß der Behälter (2) in zwei Kammern unterteilt ist, von denen jede ihre eigene unabhängige Leitung (10), ihr eigenes unabhängiges Ventil (3) und das eigene unabhängige Nasenelement (9) versorgt.

10. Spender nach Anspruch 7, 8 oder 9, dadurch gekennzeichnet, daß das Ventil (3) oder die Ventile durch Betätigungsmittel betätigt werden, welche an den Rand der Nasen(scheide)wand anstoßen.

11. Spender nach einem der vorstehenden Ansprüche, dadurch gekennzeichnet, daß der Spender als wegwerfbare Vorrichtung ausgestaltet ist.

12. Spender nach Anspruch 11, dadurch gekennzeichnet, daß der Spender nach der Verwendung seine Erscheinungsform irreversibel verändert.

13. Spender nach Anspruch 12, dadurch gekennzeichnet, daß der Spender mit einer Hülle (4) versehen ist, welche nach dem Entfernen nicht zurücksetzbar ist.

## Revendications

1. Un distributeur pour l'administration intranasale d'une ou plusieurs substances biologiquement actives, par exemple des doses de médicament, comprenant une ou deux pièces nasales (9) et caractérisé en ce qu'il est actionné par contact nasal, lorsque la ou les pièce(s) nasale(s) (9) est(sont) insérée(s) dans une narine ou dans les narines d'un mammifère, par exemple d'un être humain.

2. Un distributeur selon la revendication 1, caractérisé en ce qu'il comporte en outre un récipient (2) contenant un agent propulseur sous pression, par exemple de l'air atmosphérique, au moins un conduit (10) reliant la ou les pièce(s) nasale(s) au récipient (2), au moins une valve (3) obturant le passage du récipient (2) vers la ou les pièce(s) nasale(s) (9), et des moyens pour ouvrir la valve par contact nasal, la substance biologiquement active étant contenue dans l'agent propulseur ou dans le passage à partir du récipient et à travers le conduit dans la pièce nasale.

3. Un distributeur selon la revendication 2, caractérisé en ce que la ou les valve(s) (3) sont d'un type bistable, obturé en passant à l'état complètement ouvert lorsqu'elle(s) est (sont) actionnée(s).

4. Un distributeur selon l'une quelconque des revendications précédentes, caractérisé en ce que la ou les pièce(s) nasale(s) (9) est (sont) flexible(s) pour s'adapter elle(s)-même(s) au nez du patient.

5. Un distributeur selon l'une quelconque des revendications précédentes, caractérisé en ce que les substances biologiquement actives sont liquides ou dissoutes ou sous forme d'une poudre, et/ou d'une suspension, et sont placées dans le conduit (10) ou dans la pièce nasale (9).

6. Un distributeur selon l'une quelconque des revendications 1 à 4, caractérisé en ce que les substances biologiquement actives apparaissent comme un solide, sous la forme d'une poudre et/ou d'une suspension placée dans le conduit (10) et/ou dans la pièce nasale (9), et en ce qu'un solvant constitue une partie du propulseur ou bien est placé dans ce conduit (10) ou dans la pièce nasale (9), en amont par rapport à la poudre ou à la suspension, et est séparé de cette poudre ou de cette suspension.

7. Un distributeur selon l'une quelconque des revendications précédentes, caractérisé en ce qu'il comporte deux pièces nasales (9).

8. Un distributeur selon la revendication 7, caractérisé en ce que les pièces nasales (9) comportent chacune leur propre conduit indépendant (10) et leur propre valve indépendante (3), les valves étant conçues pour s'ouvrir simultanément.

9. Un distributeur selon la revendication 7, caractérisé en ce que le récipient (2) est divisé en deux compartiments alimentant chacun son propre conduit indépendant (10), sa propre valve indpendante (3) et sa propre pièce nasale indépendante (9).

10. Un distributeur selon la revendication 7, 8 ou 9, caractérisé en ce que la ou les valve(s) (3) est (sont) actionnée(s) par des moyens d'actionnement venant en butée sur le bord du septum des narines.

11. Un distributeur selon l'une quelconque des revendications précédentes, caractérisé en ce qu'il est réalisé sous la forme d'un dispositif jetable.

12. Un distributeur selon la revendication 11, caractérisé en ce qu'il est réalisé pour changer d'apparence, de façon irréversible, lorsqu'il est utilisé.

13. Un distributeur selon la revendication 12, caractérisé en ce qu'il est muni d'un couvercle (4) qui ne peut pas être remis en place lorsqu'il est enlevé.
